# EUROPÄISCHE PATENTSCHRIFT

(11) **EP 1 442 001 B1**
(45) Veröffentlichungstag und Bekanntmachung des Hinweises auf die Patenterteilung: **13.08.2008**
(21) Anmeldenummer: 02774764.1
(22) Anmeldetag: 02.11.2002
(51) Int. Cl.: C07C 7/10

(54) **VERFAHREN ZUR ENTFERNUNG POLARISIERBARER VERUNREINIGUNGEN AUS KOHLENWASSERSTOFFEN MIT IONISCHEN FLÜSSIGKEITEN**
METHOD FOR ELIMINATING POLARIZABLE IMPURITIES FROM HYDROCARBONS AND HYDROCARBON MIXTURES
PROCEDE D'ELIMINATION D'IMPURETES POLARISABLES CONTENUES DANS DES HYDROCARBURES ET MELANGES D'HYDROCARBURES

(30) Priorität: 08.11.2001 DE 10155281
(43) Veröffentlichungstag der Anmeldung: 04.08.2004
(73) Patentinhaber: Merck Patent GmbH, 64293 Darmstadt (DE)
(72) Erfinder: WASSERSCHEID, Peter, 50829 Köln (DE); BÖSMANN, Andreas, 52068 Aachen (DE); JESS, Andreas, 95447 Bayreuth (DE); DATSEVICH, Leonid, 95445 Bayreuth (DE); SCHMITZ, Christoph, 41363 Jüchen (DE); WENDT, Andrea, 61462 Königstein (DE)
(86) Internationale Anmeldenummer: PCT/EP2002/012239
(87) Internationale Veröffentlichungsnummer: WO 2003/037835

(56) Entgegenhaltungen:
- WO-A-01/40150
- WO-A-01/55060
- DE-C- 767 891

## Beschreibung

Die Erfindung bezieht sich auf ein Verfahren zur Entfernung von polarisierbaren Verunreinigungen aus Kohlenwasserstoffen und Kohlenwasserstoffgemischen unter Verwendung von ionischen Flüssigkeiten als Extraktionsmedium mit dem Ziel den Verunreinigungsgrad des Kohlenwasserstoffs bzw. Kohlenwasserstoffgemisches auf ein niedriges oder sehr niedriges Niveau abzusenken.

### Stand der Technik und Problemstellung

Die Abtrennung polarisierbarer Verunreinigungen aus Kohlenwasserstoffen oder Kohlenwasserstoffgemischen - bis in den ppm-Bereich hinein - ist technisch von großem Interesse und erweist sich häufig als problematisch. Die Bedeutung solcher Reinigungsverfahren beruht auf der Tatsache, dass viele polarisierbare Verunreinigungen in Kohlenwasserstoffen bzw. Kohlenwasserstoffgemischen deren technische Verwendbarkeit einschränken, da sie sich entweder störend auf den eigentlichen technischen Prozess auswirken oder zu aus umweltpolitischer Sicht unerwünschten Nebenprodukten führen. Wichtige Beispiele hierfür sind die Abtrennung von Schwefel- und Stickstoffkomponenten aus Treibstoffen zur Reduktion der SO₂-Emissionen oder die Reduzierung chlorhaltiger Verbindungen aus Motorölen zur Vermeidung von Korrosionsproblemen.

Die Tiefentschwefelung von Kraftstoffen zur Erreichung sehr kleiner Schwefelgehalte (> 50ppm) stellt ein großes Problem bei der Treibstoffrherstellung dar. Die gesetzlichen Rahmenbedingungen sehen zur Zeit eine Absenkung des Schwefelgehaltes in Otto- und Dieselkraftstoffen auf 50 ppm für das Jahr 2005 Innerhalb der Europäischen Union vor. Deutsche Regierungsvorschläge zielen bereits auf eine Absenkung auf 10 ppm im Jahre 2003 (W. Bonse-Geuking, Erdöl Erdgas Kohle, 116, 9 (2000) 407). Nach dem heutigen Stand der Technik erfolgt in einer Raffinerie die Abtrennung von schwefelhaltigen Verbindungen durch katalytische Hydrierung (J. Pachano, J. Guitian, O. Rodriguez, J.H. Krasuk (Intevep, S.A.), US-A-4752376 (1988), Jr. Hensley, L. Albert, LM. Quick (Standard Oil Company), US-A-4212729 (1980), S.B. Alpert, R.H. Wolk, M.C. Chervenak, G. Nongbri (Hydrocarbon Research, Inc.), US-A-3725251 (1971), G.R. Wilson (Gulf Research & Development Company), US-A-3898155 (1975), Y. Fukui, Y. Shiroto, M. Ando, Y. Homma (Chiyoda Chemical Engineering & Construction Co., Ltd.), US-A-4166026 (1979), R.H. Fischer, J. Ciric, T.E. Whyte (Mobil Oil Corporation), US-A-3867282 (1975), J.G. Gatsis (Universal Oil Products Company), US-A-3859199 (1975), L.K. Riley, W.H. Sawyer (Esso Research and Engineering Company), US-A-3770617 (1973), C.E. Adams, W.T. House (Esso Research and Engineering Company), US3668116 (1972)). Die Abtrennung des entstehenden Schwefeiwasserstoffes wird durch Aminwäscher erreicht (W.W. Kensell, M.P. Quinlan, The M.W. Kellogg Company Refinery Sulfur Management, in: R.A. Meyers (Ed.), Handbook of Petroleum Refining Processes, New York, San Francisco, Washington, D.C., Auckland, Bogotá, Caracas, Lisbon, London, Madrid, Mexico City, Milan, Montreal, New Dehli, San Juan, Singapore, Sydney, Tokyo, Toronto: McGraw-Hill, 1996, 11.3). Ein weiteres Verfahren ist der UOP Merox Prozess. Hierbei werden die im Kraftstoff vorhandenen Mercaptane in Anwesenheit eines metallorganischen Katalysators bei niedrigen Temperaturen in alkalischer Umgebung mit Sauerstoff zu Disulfiden umgesetzt (D.L. Holbrook, UOP Merox Process, in: R.A. Meyers (Ed.), Handbook of Petroleum Refining Processes, New York, San Francisco, Washington, D.C., Auckland, Bogotá, Caracas, Lisabon, London, Madrid, Mexico City, Milan, Montreal, New Dehli, San Juan, Singapore, Sydney, Tokyo, Toronto: McGraw-Hill, 1996, 11.29).

Halogenierte Verunreinigungen in Kohlenwasserstoffen und Kohlenwasserstoffgemischen verursachen in der Technik Korrosionsprobleme, weshalb ihr Gehalt in der Regel bis in den Bereich weniger ppm vermindert werden muß. Technisch erreicht man dies durch katalytische Hydrierung oder elektrolytische Dehalogenierung (G. Scharfe, R.-E. Wilhelms (Bayer Aktiengeselischaft), US-A-3892818 (1975), J. Langhoff, A. Jankowski, K.-D. Dohms (Ruhrkohle AG), US-A-5015457 (1991), W. Dohler, R. Holighaus, K. Niemann (Veba Oel Aktiengeselischaft), US-A-4810365 (1989), F.F. Oricchio (The Badger Company, Inc.), US-A-3855347 (1974), R.W. La Hue, C.B. Hogg (Catalysts and Chemicals, Inc.), US-A-3935295 (1976), F. Rasouli, E.K. Krug (ElectroCom Gard, Ltd.), US-A-5332496 (1994), H.J. Byker (PCB Sandpiper, Inc.), US-A-4659443 (1987), J.A.F. Kitchens (Atlantic Research Corporation), US-A-4144152 (1979)).

Ionische Flüssigkeiten sind bereits seit vielen Jahren bekannt (P. Wasserscheid, W. Keim, Angew. Chem., 112 (2000) 3926; T. Welton, Chem. Rev., 99 (1999) 2071; J.D. Holbrey, K.R. Seddon, Clean Products and Processes, 1 (1999), 223). Sie sind dadurch gekennzeichnet, dass es sich um Flüssigkeiten handelt, die ausschließlich aus Ionen bestehen. Wichtige Eigenschaften sind ihre in weiten Grenzen durch Variation des Kations und/oder Anions einstellbare Löslichkeitseigenschaften und ihr unmessbar-kleiner Dampfdruck. Zahlreiche ionische Flüssigkeiten sind mit Kohlenwasserstoffen und Kohlenwasserstoffgemischen nicht vollständig mischbar d.h. es kommt zur Ausbildung von Zwei- oder Mehrphasensystemen (P. Wasserscheid, W. Keim, Angew. Chem., 112 (2000) 3926).

WO-A-01/40150 offenbart ein Verfahren zur Entfernung polarer aromatischer Verunreinigungen (z.B. aromatische Verunreinigungen, die N, S und Metalle enthalten) aus Kohlenwasserstoffgemischen, das dadurch gekennzeichnet ist, dass eine Extraktion der polaren Verunreinigungen mit ionischen Flüssigkeiten erfolgt. Als ionische Flüssigkeiten wird zum Beispiel Tetrachloraluminat als

Anion und ein Imidazolium als Kation verwendet.

Das M. Freemantle, C&EN, 2000, Seite 37-50, XP-001119183 offenbart die Verwendung von ionischen Flüssigkeiten (Hexafluorophosphat al Anion und Imidazolium als Kation) zur Entfernung von Schwefelwasserstoff und Kohlendioxid aus Erdgas.

Die Erfindung beruht auf der überraschenden Feststellung, dass durch Extraktion eines Kohlenwasserstoffs oder eines Kohlenwasserstoffgemisches mittels einer ionischen Flüssigkeit oder einem Gemisch aus ionischen Flüssigkeiten der Gehalt an polarisierbaren Verunreinigungen im Kohlenwasserstoff bzw. Kohlenwasserstoffgemisch dann erheblich reduziert werden kann, wenn die verwendete ionische Flüssigkeit eine Mischungslücke mit dem Kohlenwasserstoff bzw. dem Kohlenwasserstoffgemisch aufweist. Damit stellt die Erfindung eine neuartige, äußerst effiziente Lösung für die oben genannten Probleme zur Abtrennung von polarisierbaren Verunreinigungen aus Kohlenwasserstoffen bzw. Kohlenwasserstoffgemischen dar.

Im Einzelnen betrifft die Erfindung ein Verfahren zur Entfernung polarer Verunreinigungen aus Kohlenwasserstoffen und Kohlenwasserstoffgemischen durch eine Extraktion der polaren Verunreinigungen mit ionischen Flüssigkeiten, dadurch gekennzeichnet, dass die verwendeten ionischen Flüssigkeiten ausgewählt sind aus der Gruppe bestehend aus ionischen Flüssigkeiten der allgemeinen Formel

[A]ₙ⁺[Y]ⁿ⁻

ist wobei n = 1 oder 2 ist und
das Anion [Y]ⁿ⁻ ausgewählt ist aus der Gruppe bestehend aus Tetrafluoroborat ([BF₄]), Sulfat ([SO₄]²⁻), Fluorosulfonat, [-R'-COO]⁻, [R'-SO₃]⁻, [R'-SO₄], oder [(R'-SO₂)₂N]⁻, und R' ein linearer oder verzweigter 1 bis 16 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome substituiert sein kann, das Kation [A]⁺ ist ausgewählt aus
- quarternären Ammonium-Kationen der allgemeinen Formel

   [NR¹R²R³R]⁺,
- Phosphonium-Kationen der allgemeinen Formel

   [PR¹R²R³R]⁺,
- Imidazolium-Kationen der allgemeinen Formel 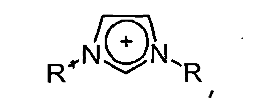
wobei der Imidazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen,
- Pyridinium-Kationen der allgemeinen Formel 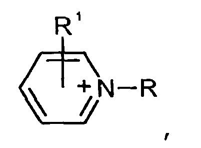
wobei der Pyridin-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen,
- Pyrazolium-Kationen der allgemeinen Formel 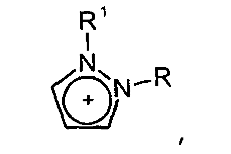
wobei der Pyrazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen,
- und Triazolium-Kationen der allgemeinen Formel 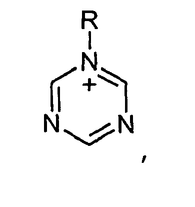
wobei der Triazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen, und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, die mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 16 Kohlenstoffatomen im Arylrest, die gegebenenfalls mit wenigstens einer C₁-C₆-Alkylgruppe und/oder einem Halogenatomen substituiert sein können; und der Rest R ausgewählt ist aus
- linearen oder verzweigten, gesättigten oder ungesättigten, aliphatischen oder alicyclischen Alkylgruppen mit 1 bis 20 Kohlenstoffatomen;
- Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 16 Kohlenstoffatomen im Arylrest, die gegebenenfalls mit wenigstens einer C₁-C₆-Alkylgruppe und/oder einem Halogenenatomen substituiert sein können.

Bevorzugte Ausgestaltungen der Erfindung ergeben sich aus den abhängigen Ansprüchen.

Zusammensetzungen, umfassend einen Kohlenwasserstoff oder ein Kohlenwasserstoffgemisch sowie wenigstens eine der oben definierten ionischen Flüssigkeiten, können als Ausgangspunkt für die Durchführung der oben genannten Extraktion sein.

Dementsprechend können die erfindungsgemäßen Zusammensetzungen neben dem Kohlenwasserstoff oder dem Kohlenwasserstoffgemisch auch die verschiedensten Substrate, Wertstoffe oder Verunreinigungen enthalten.

In einer besonderen Ausgestaltung der Erfindung können die Alkyl-, Aryl-, Arylalkyl-, Alkoxy- und Alkylaryl-Sulfonatgruppen (Anion [Y]) durch Halogenatome, insbesondere Fluor, Chlor oder Brom substituiert sein. Besonders bevorzugt sind die fluorierten, insbesondere die perfluorierten Alkyl- und obengenannten Arylsulfonate, wie das Trifluormethansulfonat (Triflat). Als nicht halogenierte Vertreter sind die Methansulfonat-, Benzolsulfonat- und die Toluolsulfonat-Gruppe zu nennen, sowie alle weiteren im Stand der Technik bekannten Sulfonat-Austrittsgruppen.

In einer weiteren Ausgestaltung der Erfindung können die Alkyl-, Aryl-, Arylalkyl-, Alkoxy- und Alkylaryl-Carboxylatgruppen durch Halogenatome, insbesondere Fluor, Chlor oder Brom substituiert sein. Besonders bevorzugt sind die fluorierten, insbesondere die perfluorierten Alkyl- und obengenannten Arylcarboxylate, wie das Trifluormethancarboxylat (Trifluoracetat; CF₃COO⁻). Als nicht halogenierte Vertreter sind die Acetat- und Benzoat-Gruppe zu nennen, sowie alle weiteren im Stand der Technik bekannten Carboxylat-Austrittsgruppen.

In bevorzugten Ausgestaltungen der Erfindung können die im Zusammenhang mit den Substituenten erwähnten C₁-C₆-Alkyl-Gruppen jeweils unabhängig voneinander durch C₂-C₄-Alkyl-Gruppen ersetzt werden. Ebenso können die im Zusammenhang mit den Substituenten erwähnten C₁-C₆-Alkoxy-Gruppen jeweils unabhängig voneinander durch C₂-C₄-Alkoxy-Gruppen ersetzt werden. In einer weiteren Alternative der Erfindung können die im Zusammenhang mit den Substituenten erwähnten C₅-C₁₆-Aryl-Gruppen jeweils unabhängig voneinander durch C₆-C₁₀-Aryl-Gruppen, die C₃-C₈-Heteroaryl-Gruppen jeweils unabhängig voneinander durch C₃-C₆-Heteroaryl-Gruppen ersetzt werden. Die Halogenatome, mit welchen die Alkyl-, Alkoxy- und Aryl-Gruppen substituiert sein können sind ausgewählt aus Fluor, Chlor, Brom und Iod, vorzugsweise Fluor, Chlor und Brom.

Ein einer bevorzugten Ausgestaltung ist der Rest R' ein linearer oder verzweigter 1 bis 8 Kohlenstoffatome enthaltender aliphatischer oder alicyclischer Alkyl- oder ein C₆-C₁₀-Aryl-, C₆-C₁₀-Aryl-C₁-C₄-alkyl- oder C₁-C₄-Alkyl-C₆-C₁₀-Ar-ylrest, der durch Halogenatome substituiert sein kann.

Die Kationen [A] sind beispielsweise ausgewählt aus Cocosalkylpentaethoxymethylammonium, Trimethylphenylammonium, Methyltrioctylarnmonium, Benzyldimethyltetradecylammonium, Tetrabutyl-phosphonium, Trihexyl(tetradecyl)phosphonium, 3-Butyl-1-methyl-imidazolium, 3-Ethyl-1-methyl-Imidazolium, N-Butylpyridinium, N-Ethylpyridinium, Diethylpyrazolium, 1-Ethyl-3-methylimidazolium, 1-Butyl-3-methylimidazolium, 1-Hexyl-3-methylimidazolium, 1-Octyl-3-methyllmidazollum, 1-Decyl-3-methylimidazolium, 1-Butyl-4-methylpyridinlum, i-Butyl-3-methylpyridinium, 1-Butyl-2-methylpyridinium, 1-Butyl-pyridinium, 1-Butyl-Methyl-Imidazolium, Nonyl-Methyl-Imidazolium, Butyl-Methyl-Imdazolium, Hexyl-Methyl-Imdazolium, Octyl-Methyl-Imdazollum, 4-Methyl-Butyl-Pyridinium, Triethylammonium, Trieethylmethylammonium, Butylmethyl-Pyridinium, Propylammonium, Methyl-Methyl-Imidazollum, Ethyl-Methyl-Imidazolium, Butyl-Methyl-Imidazolium und Butyl-Methyl-Imidazolium.

In einer Ausgestaltung des erfindungsgemäßen Verfahrens wird die ionische Flüssigkeit als alleiniges Extraktionsmedium, also frei von weiteren Extraktionsmitteln eingesetzt. Der Anteil der ionischen Flüssigkeit im Reaktionsmedium kann aber auch zwischen 0,0001 bis 99,9 Volumenprozent betragen, vorzugsweise zwischen 0,1 und 50 Volumenprozent, noch bevorzugter zwischen 0,5 und 30 Volumenprozent, bezogen auf die Gesamtmenge des Reaktionsmediums.

Das Reaktionsmedium kann zusätzlich zu der ionischen Flüssigkeit noch ein oder mehrere weitere Extraktionsmittel enthalten. Diese können ausgewählt sein aus der Gruppe bestehend aus Wasser, Puffer-Lösungen (pH 2 bis 10, vorzugsweise 5 bis 8) und organischen Lösungsmitteln. Einsetzbare organische Lösungsmittel sind mit Wasser mischbar oder nicht mit Wasser mischbar. Beispielhaft seien als organische Lösungsmittel Methyl-tert.-butylether, Toluol, Hexan, Heptan, tert.-Butanol, Glycole, Polyalkylenglycole genannt. Im übrigen kommen aber grundsätzlich alle herkömmlichen, auf dem Gebiet der Extraktion bekannten Extraktionsmittel in Frage.

Das zu extrahierende Medium wird durch ein Kohlenwasserstoff oder durch ein Kohlenwasserstoffgemisch dargestellt. Exemplarisch sind die Abtrennung von Schwefel- und Stickstoffkomponenten aus Treibstoffen zur Reduktion der SO2-Emissionen oder die Reduzierung chlorhaltiger Verbindungen aus Motorölen zur Vermeidung von Korrosionsproblemenzu nennen.

Als zu extrahierende Stoffe kommen grundsätzlich sämtliche polarisierbaren Verbindungen in Frage. Beispielhaft sind hier schwefelhaltige und halogenhaltige Verbindungen zu nennen.

Erfindungsgemäß wurde gefunden, das die Extraktion von schwefelhaltigen Verunreinigungen aus Kohlenwasserstoffen bzw. Kohlenwasserstoffgemischen mit ionischen Flüssigkeiten oder einem Gemisch aus ionischen Flüssigkeiten signifikante Vorteile im Vergleich zum nach heutigem Stand der Technik verwendeten Hydrierverfahren bietet. Die Vorteil beziehen sich auf Minimierung des Wasserstoffverbrauchs, Senkung der Betriebs- und Investitionskosten bis zu 50% und Erreichung, im Gegensatz zum Stand der Technik, von absoluten Minimalgrenzen des zu extrahierenden Mediums.

In Anwendung des Verfahrens gemäß dieser Erfindung tritt beispielsweise bei der Entschwefelung von Dieselkraftstoff die Extraktion der schwefelhaltigen Verunreinigungen mittels ionischer Flüssigkeiten an die Stelle der heute praktizierten Hydrierungsreaktionen. Dabei ist das Verfahren gemäß dieser Erfindung nicht auf bestimmte schwefelhaltige Verbindungsklassen beschränkt, sondern reduziert universell bei der Aufarbeitung von Erdölfraktionen den Gehalt an schwefelhaltigen Verunreinigungen. Durch mehrstufiges Extrahieren kann dabei der Restschwefelgehalt bis unter die Nachweisgrenze der verwendeten Analytik (< 1ppm) abgesenkt werden. Damit stellt das Verfahren gemäß dieser Erfindung eine neuartige Möglichkeit dar, den Schwefelgehalt in unterschiedlichen Kraftstoffen deutlich zu verringern und klar unter die in Zukunft geforderten gesetzlichen Vorgaben von 50 ppm zu bringen.

Das erfindungsgemäße Verfahren kann bei Temperaturen von -150 °C bis 500 °C, vorzugsweise in einem Temperaturbereich von -25 °C bis 200 °C, besonders bevorzugt in einem Temperaturbereich von 0°C bis 150°C durchgeführt werden.

Das Verfahren kann in einem Temperaturbereich stattfinden, in dem die ionische Flüssigkeiten entweder in flüssiger oder fester Form vorliegt.

In bevorzugten Ausgestaltungen der Erfindung kann die Extraktion mittels einer Mixer-Settier-Antage, Bodenkolonne, Blasensäulekolonne , Packungskolonne, Füllkörperkolonne, Tellerabscheider, Rotating Disc Kolonne oder Schwingplattenkolonne durchgeführt werden.

Eine Reextraktion der Schwefelverbindung aus der ionischen Flüssigkeit im Sinne einer Regenerierung der als Extraktionsmittel verwendeten ionischen Flüssigkeit ist ebenfalls möglich. Hierfür eignen sich insbesondere Medien, die ebenfalls eine Mischungslücke mit der ionischen Flüssigkeit besitzen aber sehr leichtflüchtig sind. Die Abtrennung der extrahierten Substrate aus der ionischen Flüssigkeit kann durch Extraktion, mit oder ohne vorhergehender Hydrierung oder Oxidation, mit einem Kohlenwasserstoff oder einem Kohlenwasserstoffgemisch, einer organische Verbindungen oder einem Gemisch organischer Verbindungen, flüssigem Kohlendioxid, überkritischem Kohlendioxid, flüssigem Propan oder überkritischem Propan mit anschließender Destillation, Wasserdampfdestillation, Sublimation oder Absorption an einem Absorbens (z.B. Aktivkohle oder Zeolith) erfolgen.

Die durch Reextraktion gereinigte ionische Flüssigkeit kann wieder in den Entschwefelungsprozess zurückgeführt werden.

Bei der Enthalogenierung umgeht das neuartige Verfahren gemäß dieser Erfindung die klassischen Nachteile der konventionellen Enthalogenierung durch Hydrierung (hoher Druck, hohe Temperaturen, Freisetzung des korrosiven HCl-Gas).

Die Erfindung wird durch die nachfolgenden Beispiele näher beschrieben ohne jedoch auf diese beschränkt zu sein.

### Beispiele

### 1. Entschwefelung von Dibenzothiophen in n-Dodecan

Am Beispiel der Entschwefelung der Modellkomponente Dibenzothiophen gelöst in n-Dodecan soll die Leistungsfähigkeit der Erfindung demonstriert werden. Abb. 1 zeigt die Strukturen einiger der verwendeten ionischen Flüssigkeiten und der dadurch erzielten Schwefelgehalte nach einmaliger Extraktion ausgehend von einem Schwefelgehalt von 500 ppm. Das Dibenzothiophen dient in diesem Fall als Modellkomponente für eine nach klassischem Hydrierungsverfahren schwer zu entschwefelnde Verbindung.

### Beispiele für verwendete ionische Flüssigkeiten

(a) 1-Butyl-3-methylimidazoliumtetrachloroaluminat; (b) 1-Ethyl-3-methylimidazoliumtetrachloroaluminat; (c) Diethylcyclohexylammoniummethansulfonat / Tributylammoniummethyl-methansulfonat; (d) Dodecyltrimethylammoniumtetrachloroaluminat; (e) Trioctylmethylammoniumtetrachloroaluminat; (f) Diethylmethylcyclohexylammonium-methansulfonat / Tributylammoniummethyl-methansulfonat; (g) 1-Butyl-3-methylimidazollum-BTA; (h) 1,3-Dimethylimidazolium-methansulfonat
(a), (b), (d), (e) = nicht nach der Erfindung

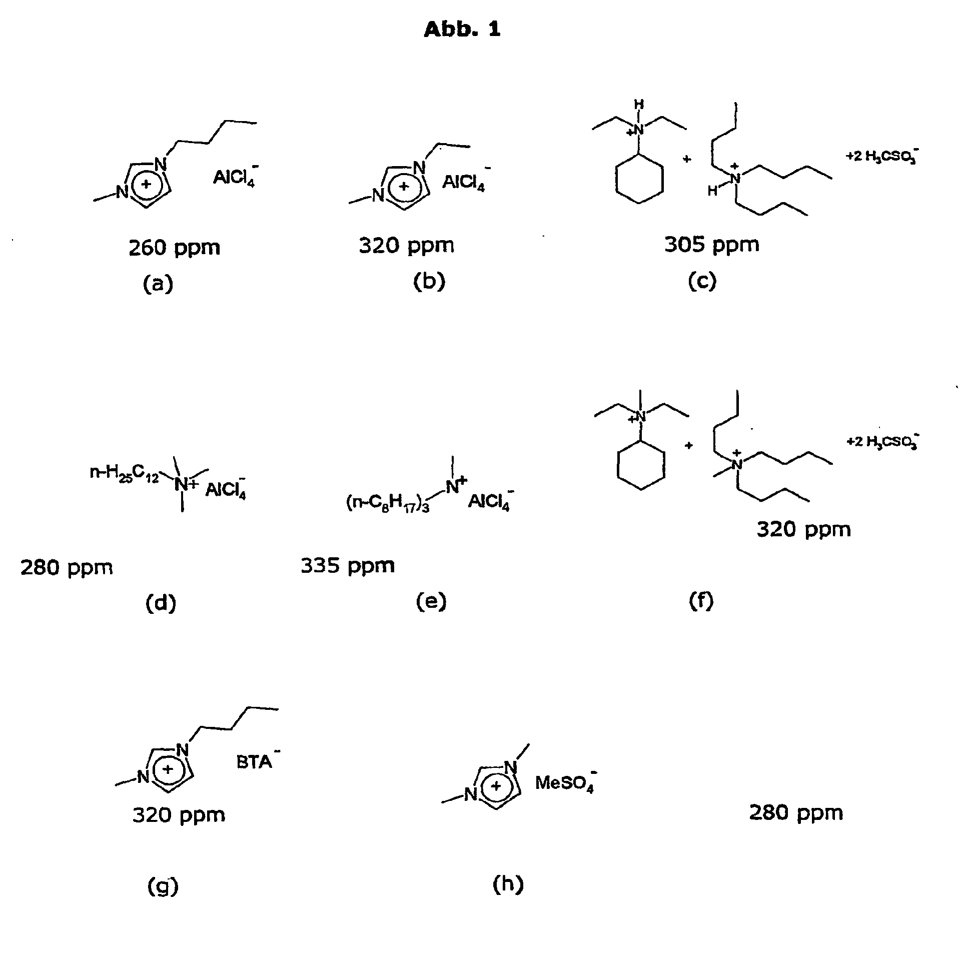

Die ionische Flüssigkeit und das Modellöl werden im Massenverhältnis 1 zu 5 für 15 Minuten bei Raumtemperatur bzw. bei 60°C im Falle für (c) und (f), da es sich bei diesen bei Raumtemperatur um Feststoffe handelt, intensiv gerührt. Danach wird das Modellöl aus dem Zweiphasengemisch abgetrennt und der Schwefelgehalt mittels Verbrennungsanalyse bestimmt.

### 2. Mehrstufige Entschwefelung von Dibenzothiophen in n-Dodecan

Hier wird gezeigt, dass mittels einer mehrstufigen Extraktion der Modellkomponente Dibenzothiophen mit ionischen Flüssigkeiten eine weitere deutliche Verringerung des Schwefelgehaltes erreicht werden kann. Weiterhin wird gezeigt, dass die unterschiedlichen ionischen Flüssigkeiten zum Teil deutlich unterschiedlich gut geeignet sind. Das Verfahren selber ist das gleiche, wie in Beispiel 1. Das einmal entschwefelte Modellöl wird jedoch in einer zweiten, dritten etc. Stufe jeweils wiederum mit frischer ionischer Flüssigkeit umgesetzt. Die Ergebnisse sind in Tabelle 1 dargestellt.

**Tabelle 1**

| Ergebnisse mehrstufiger Extraktionen von Modellöl (500 ppm) mit ionischen Flüssigkeiten | | | | |
|---|---|---|---|---|
| Verbindung | Schwefelgehalt [ppm] Stufe | | | |
| | 1 | 2 | 3 | 4 |
| (a)* | 260 | 120 | 55 | 25 |
| (c) | 305 | 195 | 120 | 65 |
| (f) | 335 | 210 | 130 | 85 |
| (h) | 450 | 420 | 405 | |

| | | | | |
|---|---|---|---|---|
| *) nicht nach der Erfindung | | | | |

### 3. Entschwefelung von realen Kraftstoffen

Die folgenden Beispiele zeigen, dass eine Übertragung der Versuche mit der Modellkomponente Dibenzothiophen zur Entschwefelung mit ionischen Flüssigkeiten auf komplexe reale Systeme möglich ist. Hierzu wird genauso vorgegangen wie in Beispiel 2. Als Kraftstoff wird beispielhaft eine vorentschwefelte Dieselfraktion mit einem Schwefelgehalt von 375 ppm verwendet. Einige Ergebnisse sind in Tabelle 2 zusammengefasst.

**Tabelle 2**

| Ergebnisse mehrstufiger Extraktionen von Dieselkraftstoff (375 ppm) mit ionischen Flüssigkeiten | | | | | |
|---|---|---|---|---|---|
| Verbindung | Schwefelgehalt [ppm] Stufe | | | | |
| | 1 | 2 | 3 | 4 | 8 |
| (a)* | 220 | 160 | 130 | 100 | 40 |
| (c) | 325 | 290 | 250 | 225 | |
| (f) | 330 | 300 | | | |

| | | | | | |
|---|---|---|---|---|---|
| *) nicht nach der Erfindung | | | | | |

## Patentansprüche

1. Verfahren zur Entfernung polarer Verunreinigungen aus Kohlenwasserstoffen und Kohlenwasserstoffgemischen durch eine Extraktion der polaren Verunreinigungen mit ionischen Flüssigkeiten, **dadurch gekennzeichnet, dass** die verwendeten ionischen Flüssigkeiten ausgewählt sind aus der Gruppe bestehend aus ionischen Flüssigkeiten der allgemeinen Formel
[A]ₙ⁺[Y]ⁿ⁻
ist wobei n = 1 oder 2 ist und
das Anion [Y]ⁿ⁻ ausgewählt ist aus der Gruppe bestehend aus Tetrafluoroborat ([BF₄]), Sulfat ([SO₄]²⁻), Fluorosulfonat, [R'-COO]⁻, [R'-SO₃]⁻, [R'-SO₄], oder [(R'-SO₂)₂N]⁻, und R' ein linearer oder verzweigter 1 bis 16 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome substituiert sein kann, das Kation [A]⁺ ist ausgewählt aus
- quarternären Ammonium-Kationen der allgemeinen Formel
[NR¹R²R³R]⁺,
- Phosphonium-Kationen der allgemeinen Formel
[PR¹R²R³R]⁺,
- Imidazolium-Kationen der allgemeinen Formel 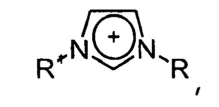 wobei der Imidazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen,
- Pyridinium-Kationen der allgemeinen Formel 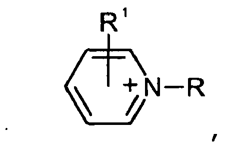 wobei der Pyridin-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen,
- Pyrazolium-Kationen der allgemeinen Formel 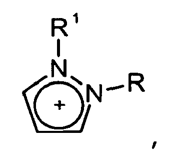 wobei der Pyrazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen,
- und Triazolium-Kationen der allgemeinen Formel 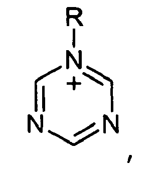 wobei der Triazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₆-Aryl- oder C₅-C₁₆-Aryl-C₁-C₆-Alkylgruppen,
und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, die mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 16 Kohlenstoffatomen im Arylrest, die gegebenenfalls mit wenigstens einer C₁-C₆-Alkylgruppe und/oder einem Halogenatomen substituiert sein können;
und der Rest R ausgewählt ist aus
- linearen oder verzweigten, gesättigten oder ungesättigten, aliphatischen oder alicyclischen Alkylgruppen mit 1 bis 20 Kohlenstoffatomen;
- Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 16 Kohlenstoffatomen im Arylrest, die gegebenenfalls mit wenigstens einer C₁-C₆-Alkylgruppe und/oder einem Halogenenatomen substituiert sein können.

2. Verfahren nach einem der vorangehenden Ansprüche, **dadurch gekennzeichnet ist, dass** es sich bei Substraten, die entfernt werden sollen um schwefelhaltige oder halogenhaltige Verbindungen handelt.

3. Verfahren nach einem oder mehreren der vorangehenden Ansprüche,
**dadurch gekennzeichnet ist, dass** die Extraktion in einem Temperaturbereich von -150°C bis 500°C, vorteilhafter im Bereich -25°C bis 200°C und besonders vorteilhaft von 0°C bis 150°C durchgeführt wird.

4. Verfahren nach einem oder mehreren der vorangehenden Ansprüche,
**dadurch gekennzeichnet ist, dass** sowohl Extraktion als auch Abtrennung vom Kohlenwasserstoff oder Kohlenwasserstoffgemisch in einem Temperaturbereich stattfinden, in dem die ionische Flüssigkeit in fester oder flüssiger Form vorliegt.

5. Verfahren nach einem oder mehreren der vorangehenden Ansprüche,
**dadurch gekennzeichnet ist, dass** die Menge zugesetzter ionischer Flüssigkeit einen Massenanteil im Bereich 0,01 bis 99%, vorzugsweise 0,1 bis 50% und am geeignetsten 0,5 bis 30% hat.

6. Verfahren nach einem oder mehreren der vorangehenden Ansprüche,
**dadurch gekennzeichnet ist, dass** die Extraktion in einem Mixer-Settler-Anlage, Bodenkolonne, Blasensäulekolonne, Packungskolonne, Füllkörperkolonne, Tellerabscheider, Rotating Disc Kolonne oder Schwingplattenkolonne stattfindet.

7. Verfahren nach einem oder mehreren der vorangehenden Ansprüche,
**dadurch gekennzeichnet ist, dass** die Abtrennung der extrahierten Verunreinigungen aus der ionischen Flüssigkeit, mit oder ohne vorhergehende Hydrierung oder Oxidation, mittels einer Extraktion mit einem Kohlenwasserstoff oder einem Kohlenwasserstoffgemisch, einer organische Verbindungen oder einem Gemisch organischer Verbindungen, flüssigem Kohlendioxid, überkritischem Kohlendioxid, flüssigem Propan oder überkritischem Propan oder durch Destillation, Wasserdampfdestillation, Sublimation oder Absorption an einem Absorbens (z.B. Aktivkohle oder Zeolith) erfolgt.

## Claims

1. Process for the removal of polar impurities from hydrocarbons and hydrocarbon mixtures by extraction of the polar impurities using ionic liquids, **characterised in that** the ionic liquids used are selected from the group consisting of ionic liquids of the general formula
[A]ₙ⁺[Y]ⁿ⁻
where n = 1 or 2 and
the anion [Y]ⁿ⁻ is selected from the group consisting of tetrafluoroborate ([BF₄]), sulfate ([SO₄]²⁻), fluorosulfonate, [R'-COO]⁻, [R'-SO₃]⁻, [R'-SO₄] or [(R'-SO₂)₂N]⁻, and R' is a linear or branched aliphatic or alicyclic alkyl radical containing 1 to 16 carbon atoms or a C₅-C₁₈-aryl, C₅-C₁₈-aryl-C₁-C₆-alkyl or C₁-C₆-alkyl-C₅-C₁₈-aryl radical, which may be substituted by halogen atoms,
the cation [A]⁺ is selected from
- quaternary ammonium cations of the general formula
[NR¹R²R³R]⁺,
- phosphonium cations of the general formula
[PR¹R²R³R]⁺,
- imidazolium cations of the general formula 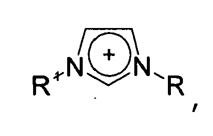
where the imidazole ring may be substituted by at least one group selected from C₁-C₆-alkyl, C₁-C₆-alkoxy, C₁-C₆-aminoalkyl, C₅-C₁₆-aryl or C₅-C₁₆-aryl-C₁-C₆-alkyl groups,
- pyridinium cations of the general formula 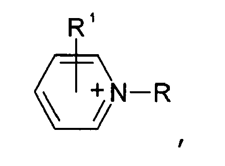
where the pyridine ring may be substituted by at least one group selected from C₁-C₆-alkyl, C₁-C₆-alkoxy, C₁-C₆-aminoalkyl, C₅-C₁₆-aryl or C₅-C₁₆-aryl-C₁-C₆-alkyl groups,
- pyrazolium cations of the general formula 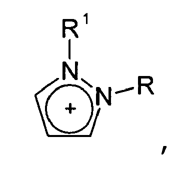
where the pyrazole ring may be substituted by at least one group selected from C₁-C₆-alkyl, C₁-C₆-alkoxy, C₁-C₆-aminoalkyl, C₅-C₁₆-aryl or C₅-C₁₆-aryl-C₁-C₆-alkyl groups,
- and triazolium cations of the general formula 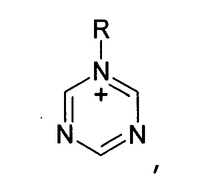 where the triazole ring may be substituted by at least one group selected from C₁-C₆-alkyl, C₁-C₆-alkoxy, C₁-C₆-aminoalkyl, C₅-C₁₆-aryl or C₅-C₁₆-aryl-C₁-C₆-alkyl groups,
and the radicals R¹, R², R³ are selected, independently of one another, from the group consisting of
- hydrogen;
- linear or branched, saturated or unsaturated, aliphatic or alicyclic alkyl groups having 1 to 20 carbon atoms;
- heteroaryl, heteroaryl-C₁-C₆-alkyl groups having 3 to 8 carbon atoms in the heteroaryl radical and at least one heteroatom selected from N, O and S, which may be substituted by at least one group selected from C₁-C₆-alkyl groups and/or halogen atoms;
- aryl, aryl-C₁-C₆-alkyl groups having 5 to 16 carbon atoms in the aryl radical, which may optionally be substituted by at least one C₁-C₆-alkyl group and/or halogen atom;
and the radical R is selected from
- linear or branched, saturated or unsaturated, aliphatic or alicyclic alkyl groups having 1 to 20 carbon atoms;
- heteroaryl-C₁-C₆-alkyl groups having 3 to 8 carbon atoms in the aryl radical and at least one heteroatom selected from N, O and S, which may be substituted by at least one C₁-C₆-alkyl group and/or halogen atom;
- aryl-C₁-C₆-alkyl groups having 5 to 16 carbon atoms in the aryl radical, which may optionally be substituted by at least one C₁-C₆-alkyl group and/or halogen atom.

2. Process according to one of the preceding claims, **characterised in that** the substances to be removed are sulfur-containing or halogen-containing compounds.

3. Process according to one or more of the preceding claims, **characterised in that** the extraction is carried out in a temperature range from -150°C to 500°C, more advantageously in the range from -25°C to 200°C and particularly advantageously from 0°C to 150°C.

4. Process according to one or more of the preceding claims, **characterised in that** both extraction and separation of the hydrocarbon or hydrocarbon mixture are carried out in a temperature range in which the ionic liquid is in solid or liquid form.

5. Process according to one or more of the preceding claims, **characterised in that** the amount of added ionic liquid has a proportion by weight in the range 0.01 to 99%, preferably 0.1 to 50% and most suitably 0.5 to 30%.

6. Process according to one or more of the preceding claims, **characterised in that** the extraction is carried out in a mixer-settler unit, tray column, bubble column, column with regular packing, column with irregular packing, plate separator, rotating disc column or vibrating plate column.

7. Process according to one or more of the preceding claims, **characterised in that** the separation of the extracted impurities from the ionic liquid is carried out, with or without prior hydrogenation or oxidation, by means of extraction with a hydrocarbon or hydrocarbon mixture, an organic compound or a mixture of organic compounds, liquid carbon dioxide, supercritical carbon dioxide, liquid propane or supercritical propane or by distillation, steam distillation, sublimation or absorption on an absorbent (for example activated carbon or zeolite).

## Revendications

1. Procédé d'élimination d'impuretés polaires à partir d'hydrocarbures et de mélanges d'hydrocarbures par extraction des impuretés polaires à l'aide de liquides ioniques, **caractérisé en ce que** les liquides ioniques utilisés sont choisis parmi le groupe constitué par les liquides ioniques de formule générale
[A]ₙ⁺[Y]ⁿ⁻
dans laquelle n = 1 ou 2 et
l'anion [Y]ⁿ⁻ est choisi parmi le groupe constitué par tétrafluoroborate ([BF₄]), sulfate ([SO₄]²⁻), fluorosulfonate, [R'-COO]⁻, [R'-SO₃]⁻, [R'-SO₄] ou [(R'-SO₂)₂N]⁻, et R' est un radical alkyle aliphatique ou alicyclique, linéaire ou ramifié, contenant de 1 à 16 atomes de carbone ou un radical C₅-C₁₈-aryle, C₅-C₁₈-aryl-C₁-C₆-alkyle ou C₁-C₆-alkyl-C₅-C₁₈-aryle, pouvant être substitué par des atomes d'halogène, le cation [A]⁺ est choisi parmi
- des cations ammonium quaternaire de formule générale
[NR¹R²R³R]⁺,
- des cations phosphonium de formule générale
[PR¹R²R³R]⁺,
- des cations imidazolium de formule générale 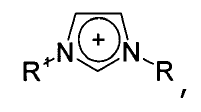
dans laquelle le cycle imidazolique peut être substitué par au moins un groupement choisi parmi les groupements C₁-C₆-alkyle, C₁-C₆-alcoxy, C₁-C₆-aminoalkyle, C₅-C₁₆-aryle ou C₅-C₁₆-aryl-C₁-C₆-alkyle,
- des cations pyridinium de formule générale 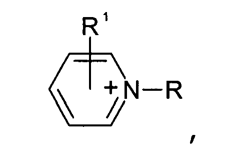
dans laquelle le cycle pyridinique peut être substitué par au moins un groupement choisi parmi les groupements C₁-C₆-alkyle, C₁-C₆-alcoxy, C₁-C₆-aminoalkyle, C₅-C₁₆-aryle ou C₅-C₁₆-aryl-C₁-C₆-alkyle,
- des cations pyrazolium de formule générale 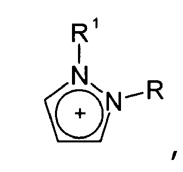
dans laquelle le cycle pyrazolique peut être substitué par au moins un groupement choisi parmi les groupements C₁-C₆-alkyle, C₁-C₆-alcoxy, C₁-C₆-aminoalkyle, C₅-C₁₆-aryle ou C₅-C₁₆-aryl-C₁-C₆-alkyle,
- et des cations triazolium de formule générale 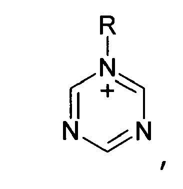 dans laquelle le cycle triazolique peut être substitué par au moins un groupement choisi parmi les groupements C₁-C₆-alkyle, C₁-C₆-alcoxy, C₁-C₆-aminoalkyle, C₅-C₁₆-aryle ou C₅-C₁₆-aryl-C₁-C₆-alkyle,
et les radicaux R¹, R², R³ sont choisis, indépendamment les uns des autres, parmi le groupe constitué par
- l'hydrogène ;
- des groupements alkyle aliphatiques ou alicycliques, saturés ou insaturés, linéaires ou ramifiés, ayant de 1 à 20 atomes de carbone ;
- des groupements hétéroaryle, hétéroaryl-C₁-C₆-alkyle ayant de 3 à 8 atomes de carbone dans le radical hétéroaryle et au moins un hétéroatome choisi parmi N, O et S, pouvant être substitué par au moins un groupement choisi parmi les groupements C₁-C₆-alkyle et/ou les atomes d'halogène ;
- des groupements aryle, aryl-C₁-C₆-alkyle ayant de 5 à 16 atomes de carbone dans le radical aryle, pouvant éventuellement être substitué par au moins un groupement C₁-C₆-alkyle et/ou un atome d'halogène ;
et le radical R est choisi parmi
- des groupements alkyle aliphatiques ou alicycliques, saturés ou insaturés, linéaires ou ramifiés, ayant de 1 à 20 atomes de carbone ;
- des groupements hétéroaryl-C₁-C₆-alkyle ayant de 3 à 8 atomes de carbone dans le radical aryle et au moins un hétéroatome choisi parmi N, O et S, pouvant être substitué par au moins un groupement C₁-C₆-alkyle et/ou un atome d'halogène ;
- des groupements aryl-C₁-C₆-alkyle ayant de 5 à 16 atomes de carbone dans le radical aryle, pouvant éventuellement être substitué par au moins un groupement C₁-C₆-alkyle et/ou un atome d'halogène.

2. Procédé selon la revendication précédente, **caractérisé en ce que** les substances à éliminer sont des composés soufrés ou halogénés.

3. Procédé selon l'une ou plusieurs parmi les revendications précédentes, **caractérisé en ce que** l'extraction est effectuée dans un domaine de température allant de -150°C à 500°C, plus avantageusement dans le domaine allant de -25°C à 200°C et de manière particulièrement avantageuse de 0°C à 150°C.

4. Procédé selon l'une ou plusieurs parmi les revendications précédentes, **caractérisé en ce que** l'extraction ainsi que la séparation des hydrocarbures ou du mélange d'hydrocarbures sont effectuées dans un domaine de température dans lequel le liquide ionique est sous forme solide ou liquide.

5. Procédé selon l'une ou plusieurs parmi les revendications précédentes, **caractérisé en ce que** la quantité de liquide ionique ajoutée a une proportion en poids dans la plage allant de 0,01 à 99%, préférablement de 0,1 à 50% et plus convenablement de 0,5 à 30%.

6. Procédé selon l'une ou plusieurs parmi les revendications précédentes, **caractérisé en ce que** l'extraction est effectuée dans un agitateur-décanteur, une colonne à plateaux, une colonne à bulles, une colonne à remplissage régulier, une colonne à remplissage irrégulier, un séparateur à plaques, une colonne à disques rotatifs ou une colonne à plaques vibrantes.

7. Procédé selon l'une ou plusieurs parmi les revendications précédentes, **caractérisé en ce que** la séparation des impuretés extraites à partir du liquide ionique est effectuée, avec ou sans hydrogénation ou oxydation préalable, au moyen d'une extraction par un hydrocarbure ou un mélange d'hydrocarbures, un composé organique ou un mélange de composés organiques, du dioxyde de carbone liquide, du dioxyde de carbone supercritique, du propane liquide ou du propane supercritique ou par distillation, entraînement à la vapeur, sublimation ou absorption sur un absorbant (par exemple du charbon actif ou des zéolites).
